# EUROPEAN PATENT SPECIFICATION

(11) **EP 2 029 114 B1**
(45) Date of publication and mention of the grant of the patent: **24.09.2014**
(21) Application number: 07795663.9
(22) Date of filing: 01.06.2007
(51) Int. Cl.: A61K 9/20, A61K 9/28, A61K 31/137, A61K 31/4545, A61K 9/24

(54) **Pharmaceutical compositions for sustained release of phenylephrine**
Pharmazeutische Zusammensetzung für die verzögerte Freisetzung von Phenylephrin
COMPOSITIONS PHARMACEUTIQUES POUR LA LIBÉRATION PROLONGÉE DE PHENYLEPHRINE

(30) Priority: 01.06.2006 US 810019 P
(43) Date of publication of application: 04.03.2009
(73) Proprietor: Merck Sharp & Dohme Corp., Rahway, NJ 07065-0907 (US)
(72) Inventor: ULLOA, Sergio R., 14620 Del. Tlapan (MX); VILLACAMPA RAMOS, Jose de Jesus, Mateo, 3100 Del. B. Juarez (MX); JUAREZ VARGAS, Luis Javier, 07720 Del Gustavo A. Madero (MX)
(74) Representative: Horgan, James Michael Frederic
(86) International application number: PCT/US2007/013056
(87) International publication number: WO 2007/143163

(56) References cited:
- WO-A-2005/120465
- US-A- 4 369 172
- US-A- 4 389 393
- US-A1- 2005 266 032
- US-A1- 2006 057 205

## Description

### FIELD OF THE INVENTION

The field of the invention is a sustained-release formulation for a pharmaceutical composition comprising phenylephrine as an active ingredient. The composition comprises a solid dosage form such as a tablet with hydroxypropyl methylcellulose as a major component of the tablet and phenylephrine released over a prolonged period of time by the tablet.

### BACKGROUND OF THE INVENTION

Phenylephrine and its pharmaceutically acceptable salts are recognized by those skilled in the art as safe and effective nasal decongestants for humans. Commercially-available formulations include nasal jelly, nasal drops, and nasal spray (i.e. Alconefrin® Nasal Drops or Neo-Synephrine® Nasal Jelly) as well as immediate release oral tablets or gelatin capsules (i.e. Sudafed PE^{™} or DayQuil® LiquiCaps). Due to a short half-life *in vivo*, phenylephrine and its pharmaceutically acceptable salts as currently formulated are commonly administered every four hours for the relief of nasal congestion. Thus, there is a need for sustained release formulations of phenylephrine that can be administered less frequently, for example, every eight or twelve hours.

Sustained release formulations result in a decrease in the frequency of drug administration thereby improving patient compliance. In addition, sustained drug release systems produce constant therapeutic plasma levels of active ingredients as compared to fluctuations seen when multiple doses of a conventional formulation are given. Sustained drug release systems may decrease the severity and frequency of side effects from multiple dosages or from pulsed release systems.

U.S. Pat. No. 4,792,452 discloses a tablet formulation composed of up to about 45% by weight of a pH-dependent salt of alginic acid, up to about 35% by weight of a pH-independent hydrocolloid gelling agent, binder and excipients. Release of the drug is therefore affected by the varying pH of the gastrointestinal tract. Australian patent application AU-B-56761/86 discloses examples of sustained release formulations for aspirin and theophylline including specific hydroxypropylmethylcelluloses. AU-B-56761/86 also describes phenylephrine as one of at least twenty-seven drugs or types of drugs that are typical moisture sensitive drugs.

U.S. Pat. No. 2006/0057205 discloses sustained release dosage forms comprising phenylephrine and hydroxypropyl methylcellulose.

### SUMMARY OF THE INVENTION

An object of the present invention is to provide a pharmaceutical composition comprising phenylephrine that can be orally administered on a twice-daily basis in a sustained release formulation that delivers the drug in a specific pattern for phenylephrine unaffected by the varying pH of the gastrointestinal tract. An additional object of the invention is to provide a pharmaceutical composition comprising phenylephrine compatible with incorporation of loratadine or desloratadine or other antihistamine.

Yet another object of the invention is to provide a pharmaceutical composition, in solid dosage form, that provides phenylephrine in a sustained release form either alone or in combination with another active such as one or more of an antihistamine, an analgesic, an antipyretic, a non-steroidal anti-inflammatory or a mixture of two or more of the other actives, such that the solid dosage form can be administered to an individual on a once or twice daily basis for relief of symptoms or signs associated with a cold, the flu or an allergy such as allergic rhinitis.

To meet at least one of the above objects, in one embodiment, the invention provides an extended-release pharmaceutical composition comprising phenylephrine or a pharmaceutically acceptable salt thereof in a hydrophilic polymer matrix or lattice, wherein the hydrophilic polymer matrix or lattice comprises a mixture of hydroxypropyl methylcellulose and carboxymethyl cellulose sodium salt as defined in the claims, wherein the composition further comprises one or more excipients selected from the group of a lubricant, a glidant, an antiadherent agent, and a mixture of two or more thereof, wherein the composition is a solid dosage form, and wherein a single dose of the composition administered to an individual achieves a therapeutic blood/plasma concentration of phenylephrine in the individual for about 8 to about 14 hours.

In another embodiment, the invention provides an extended-release pharmaceutical composition comprising phenylephrine or a pharmaceutically acceptable salt thereof in a hydrophilic polymer matrix or lattice, wherein the hydrophilic polymer matrix or lattice comprises a mixture of hydroxypropyl methylecellulose and carboxymethyl cellulose sodium salt as defined in the claims, wherein the composition further comprises one or more excipients selected from the group of a lubricant, a glidant, an antiadherent agent, and a mixture of two or more thereof, wherein the composition is a solid dosage form, and wherein a single dose of the composition administered to an individual achieves a therapeutic blood/plasma concentration of phenylephrine in the individual for about 8 to about 14 hours; and which composition further comprises one or more actives such as an antihistamine, an analgesic, an antipyretic, a non-steroidal anti-inflammatory or a mixture of two or more such actives. In certain preferred embodiments, the antihistamine is loratadine or desloratadine available in the composition in immediate release form. In certain embodiments of this mode of the invention, the solid dosage form comprises a bi-layer tablet comprising phenylephrine in sustained release form in one of the two layers and either loratadine or desloratadine or an analgesic, antipyretic or an NSAID in immediate release form in the other of the layers.

The invention further provides methods for preparing the extended release pharmaceutical compositions. Generally, the method of preparation comprises: preparing an extended-release formulation of phenylephrine comprising incorporating phenylephrine or a pharmaceutically acceptable salt thereof in a hydrophilic polymer matrix, wherein the hydrophilic polymer matrix comprises a mixture of hydroxypropyl methylcellulose and carboxymethyl cellulose sodium salt, combining the hydrophilic polymer matrix with one or more excipients selected from the group of a lubricant, a glidant, an antiadherent agent, and mixtures of two or more thereof, and compression-pressing the resulting mixture into a solid dosage form, wherein a single dose of the extended-release formulation achieves a therapeutic blood/plasma concentration of phenylephrine in an individual for at least about 8 to about 14 hours. In certain embodiments, the method further comprises combining one or more additional actives with the mixture prior to compression-pressing.

In one embodiment, the pharmaceutical composition comprises dexbrompheniramine maleate and phenylephrine or a pharmaceutically acceptable salt, e.g. Hydrochloride.

### BRIEF DESCRIPTION OF THE FIGURES

The invention may be understood by reference to the figure, the detailed description and the illustrative example which follows.

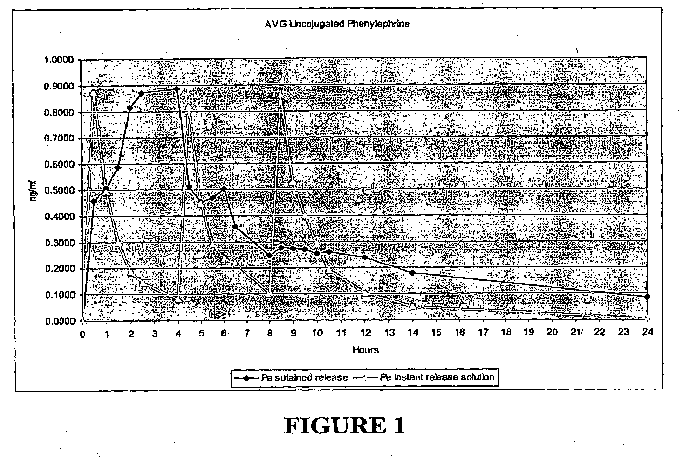

FIG. 1 illustrates the concentration of free phenylephrine (Pe) in blood/plasma after administration of a single dose of an exemplary composition according to the present invention comprising 30 mg of Pe or salt thereof in sustained release form (-•- Pe sustained release) in comparison with the concentration of Pe in blood/plasma concentration after administration of three doses of a standard immediate release formulation comprising 10mg of Pe or salt thereof (-▲- Pe instant release).

### DETAILED DESCRIPTION OF THE INVENTION

The active ingredient for the pharmaceutical compositions in extended release form according to one embodiment of the invention is phenylephrine or a pharmaceutically-acceptable salt thereof. The active ingredients for the pharmaceutical compositions according to other embodiments of the invention are phenylephrine or a pharmaceutically acceptable salt in extended release form and at least one of an antihistamine, an analgesic, anti-pyretic and a non-steroidal anti-inflammatory drug (NSAID). Pharmaceutically-acceptable salts of phenylephrine include phenylephrine hydrochloride. According to the invention, phenylephrine is released in a steady or essentially constant manner rather than in discrete bursts or pulses. Release rates for phenylephrine from the compositions according to the invention are highly dependent on the solubility parameters for the phenylephrine active ingredient. Surprisingly, results for other active ingredients, such as aspirin or theophylline, are not predictive for the particular release rate of phenylephrine in compositions according to the invention.

In a preferred embodiment, the pharmaceutical composition can include phenylephrine or a salt thereof and an antihistamine. Long-acting antihistamines selected from one or more of the group consisting of loratadine, desloratadine, azatidine, fexofenadine, terfenadine, cetirizine, astemizole, and levocabastine, or their pharmaceutically acceptable salts can be included. Preferred antihistamines include loratadine and desloratadine. Loratadine is disclosed in U.S. Pat. No. 4,282,233 as a non-sedating antihistamine useful, for example, in alleviation of seasonal allergic rhinitis symptoms such as sneezing and itching. The active metabolite of loratadine is desloratadine, which has a half-life (t^{1/2}) of approximately 15 to 19 hours. U.S. Pat. No. 5,595,997 discloses methods and compositions for treating seasonal allergic rhinitis symptoms using desloratadine. Loratadine and desloratadine are available in the form of conventional tablets that release the active agent in a conventional manner. Due to the long half life of loratadine compared to phenylephrine, the loratadine in the formulation according to the invention is preferably available for immediate release. For example, loratadine or desloratadine may be present in an immediate-release polymer coating on the surface of a solid dosage form such as a tablet. Such immediate-release coatings can be a water-soluble polymeric film. Alternatively, loratadine or desloratadine can be present in an immediate release layer composed of immediate release, with phenylephrine in a sustained release layer excipeints for tablets, forming a bi-layer tablet.

According to the invention, an amount of phenylephrine is formulated for sustained release. By sustained release is meant that the active agent becomes available for bio-absorption in the gastrointestinal tract over a prolonged period of time, such as about 1 to about 18 hours, preferably about 5 - 12 hours. The term sustained release also encompasses extended release, controlled release, or sustained delivery. The release rate of the active agent is primarily controlled by dissolution of the active agent in gastrointestinal fluid and subsequent diffusion out of the tablet independent of pH, but can also be influenced by physical processes of disintegration and erosion of the tablet. Due to the relatively short half life of phenylephrine, therapeutic blood/plasma concentrations of phenylephrine are primarily a result of release of phenylephrine from the tablet over a prolonged period of time. Pharmaceutical compositions according to the invention achieve a therapeutic blood/plasma concentration of phenylephrine in an individual for at least about 8 to about 14 hours from a single dose. In some embodiment, phenylephrine is released from the tablet to result in a therapeutic blood/plasma concentration of phenylephrine for at about 8, 9, 10, 11, 12, 13 or 14 hours from a single dose. In another preferred embodiment, phenylephrine is released from the tablet to result in a therapeutic blood/plasma concentration of phenylephrine for at least about 12 hours from a single dose, more preferably at about 12 hours. The release rate from the tablet is independent of pH, but is highly dependent on the solubility profile for phenylephrine. Active agents other than phenylephrine have different release rates than phenylephrine, and therefore are not predictive for compositions according to the invention.

As used herein, the term a "therapeutic blood/plasma concentration of phenylephrine" means concentration equal to at least about 50%, preferably at least about 80% to 125% of the AUC and/or Cₘₐₓ of phenylephrine obtained when three doses of a standard or conventional immediate release formulation comprising 10 mg of phenylephrine or salt thereof is administered to a human subject.

According to the present invention, the solid dosage form comprises one or more hydrophilic polymers. The hydrophilic polymers that may be used in the present invention include cellulose ethers such as hydroxypropyl methylcellulose, hydroxypropylcellulose, or other water soluble or swellable polymers and the like. A preferred hydrophilic polymer is hydroxypropyl methylcellulose. Examples of hydroxypropyl methylcellulose polymers that may be used in the present invention include those available from Dow Chemical Co. under the brand name Methocel™, such as, Methocel K15M, Methocel K100M, Methocel K4M and the like.

Due to the high water-solubility of phenylephrine, use of hydroxypropyl methyl cellulose by itself results in unsatisfactorily rapid diffusion and release of active agent. Combined with the above-listed hydrophilic polymer may be an additional hydrophilic polymer, such as a polyacrylate polymer or an acrylic acid copolymer or sodium carboxymethyl cellulose. A preferred polymer for use in combination with hydroxypropyl methylcellulose is carboxymethyl cellulose sodium salt. Although not intending to be limited to any particular mechanism of action, it is thought that the combination of polymers forms a matrix or lattice with active ingredient distributed within the matrix. The combination of anionic carboxymethyl cellulose sodium salt and nonionic hydroxypropyl methylcellulose may provide for stronger crosslinking of the matrix, resulting in higher viscosity and a lower diffusion rate through the matrix for phenylephrine's particular solubility profile. The claimed combination of hydroxypropyl methylcellulose and carboxymethyl cellulose sodium salt unexpectedly allows for the design of a release profile that is specific and particular for phenylephrine.

The dosage forms according to the invention are solid, and may take any customary form for oral administration, such as a tablet, a pill, a capsule, and the like. A preferred example of the solid dosage form is a compressed tablet. The tablet may form a matrix for the release of the active ingredients. Dosage forms according to the invention may further contain standard excipients, such one or more of as a disintegrant, glidant, binding agent, and antiadherent. Standard excipients include talc, stearic acid, calcium stearate, magnesium stearate, colloidal silicon dioxide, sodium lauryl sulphate, and the like. A preferred antiadherent is crosslinked insoluble polyvinylpyrrolidone available under the tradename Kollidon CL-M™ (BASF). Kollidon CL-M is preferably present in an amount from about 0.1 to about 10% by weight of the solid dosage form. In a preferred embodiment, Kollidon CL-M has a particle size distribution of about 90% of particles less than 20 microns and about 99% of particles less than 400 microns, and a specific surface area from about 3 to about 6 meters squared per gram.

It will be appreciated that the pharmaceutical compositions of the invention may also contain any one or more other additives or excipients conventionally used in the formulation of pharmaceutical compositions.

The subject to which the composition according to the invention is to be administered is not restricted. The dosage of phenylephrine varies depending on the size and age of the patient, the severity of the symptoms, and the like. The administration is preferably carried out by adjusting the dosage based on the subject's response, and is preferably administered once or twice daily. The dosage may be present in one of several different amounts of phenylephrine such as 10 mg, 15 mg, 20 mg, 25 mg and 30 mg, all of them contained in the sustained release matrix, preferably administered once or twice daily.

The dosage of antihistamine such as loratadine or desloratadine may be preset in different amounts such as 1 - 20 mg; preferably 2.5 mg, 5 mg, or 10 mg.

The dosage of analgesic and/or antipyretic such as aspirin, acetaminophen, etc. will be known to those skilled in the art and can be in the range of 80 mg to 500 mg.

The dosage of NSAID will be known to those skilled in the art and can be in the range of 80 mg to 500 mg.

### EXAMPLE

The following example is shown in order that the invention may be more readily understood.

### Formulation Example 1

A sustained-release tablet can be obtained with the following components (by weight):

| | |
|---|---|
| phenylephrine hydrochloride: | 5 to 30% |
| hydroxypropyl methylcellulose: | 20 to 70% |
| carboxymethyl cellulose sodium: | 5 to 50% |
| Kollidon CL-M: | 0.1 to 10% |
| colloidal silicon dioxide: | 0.05 to 5% |
| magnesium stearate: | 0.5 to 2% |

The above ingredients are mixed until a uniform mixture is obtained and tabletted under compression pressure using a punch to make shaped tablets. Hardness, friability, and active agent release rates are determined in the usual manner.

The above-described sustained release tablet comprising 30 mgs of Phenylephrine was administered to a human subject and the concentration of free phenylephrine in blood/plasma was determined. For comparison, three doses of 10mgs of phenylephrine in conventional immediate release formulation were administered to a human subject and the concentration of free Phenylephrine in blood/plasma was determined. Results are illustrated in FIG. 1. As shown in Fig. 1, administration of an exemplary pharmaceutical composition of the present invention comprising phenylephrine in sustained release form achieved a therapeutic blood/plasma concentration of phenylephrine for at least about 8 hours.

In one embodiment, the pharmaceutical composition comprises dexbrompheniramine maleate and phenylephrine or a pharmaceutically acceptable salt, e.g. Hydrochloride.

From the above description, one can ascertain the essential characteristics of the present invention.

## Claims

1. An extended-release pharmaceutical composition comprising phenylephrine or a pharmaceutically acceptable salt thereof in a hydrophilic polymer matrix or lattice, wherein the hydrophilic polymer matrix or lattice comprises a mixture of hydroxypropyl methylcellulose which is present in 20 to 70% by weight of the solid dosage form and carboxymethyl cellulose sodium salt which is present in 5 to 50% by weight of the solid dosage form, wherein the composition further comprises one or more excipients selected from the group of a lubricant, a glidant, an antiadherent agent, and a mixture of two or more thereof, wherein the composition is a solid dosage form, and wherein a single dose of the composition achieves a therapeutic blood/plasma concentration of phenylephrine in an individual for 8 to 14 hours.

2. The composition according to claim 1, further comprising one or more actives selected from the group consisting of an antihistamine, an analgesic, an anti-pyretic and a non-steroidal anti-inflammatory agent in immediate release form.

3. The composition according to claim 1, further comprising loratadine or desloratadine in immediate release form.

4. An extended release pharmaceutical composition comprising a bi-layer tablet, one layer comprised of a sustained release matrix comprising a mixture of hydroxypropyl methylcellulose which is present in 20 to 70% by weight of the solid dosage form and carboxymethyl cellulose sodium salt which is present in 5 to 50% by weight of the solid dosage form administered with phenylephrine or a salt thereof and the other layer comprised of an immediate release layer containing another active selected from one or more of an antihistamine, an analgesic, an antipyretic and a non-steroidal anti-inflammatory agent, wherein a single administered dose of the composition achieves a therapeutic blood/plasma concentration of phenylephrine in an individual for 8 to 14 hours.

5. The composition according to claim 4, wherein the other active is loratadine or desloratadine.

6. The composition according to claim 1, wherein the phenylephrine or a pharmaceutically acceptable salt is present in 5 to 30% by weight of the solid dosage form.

7. The composition according to claim 1, wherein the antiadherent agent is polyvinylpyrrolidone in an amount from 0.1 to 10% by weight of the solid dosage form.

8. The composition according to claim 7, wherein the polyvinylpyrrolidone has a particle size distribution of 90% of particles less than 20 microns and 99% of particles less than 400 microns.

9. The composition according to claim 7, wherein the polyvinylpyrrolidone has a specific surface area from 3 to 6 metres squared per gram.

10. The composition according to claim 1, further comprising a water-soluble polymeric film coating.

11. The composition according to claim 10, wherein the water-soluble polymeric film contains loratadine or desloratadine.

12. A method of preparing an extended release formulation of phenylephrine comprising incorporating phenylephrine or a pharmaceutically acceptable salt thereof in a hydrophilic polymer matrix, wherein the hydrophilic polymer matrix comprises a mixture of hydroxypropyl methylcellulose which is present in 20 to 70% by weight of the solid dosage form and carboxymethyl cellulose sodium salt which is present in 5 to 50% by weight of the solid dosage form, combining the hydrophilic polymer matrix with one or more excipients selected from the group of a lubricant, a glidant, an antiadherent agent, and mixtures of two or more thereof, and compression-pressing the resulting mixture into a solid dosage form, wherein a single dose of the extended-release formulation achieves a therapeutic blood/plasma concentration of phenylephrine in an individual for 8 to 14 hours.

## Patentansprüche

1. Eine pharmazeutische Zusammensetzung mit verlängerter Freisetzung, die Phenylephrin oder ein pharmazeutisch annehmbares Salz davon in einer hydrophilen Polymermatrix oder einem hydrophilen Polymergitter umfasst, wobei die hydrophile Polymermatrix oder das hydrophile Polymergitter eine Mischung aus Hydroxypropylmethylcellulose, die in 20 bis 70 Gew.-% der festen Dosisform vorliegt, und Carboxymethylcellulose-Natriumsalz, das in 5 bis 50 Gew.-% der festen Dosisform vorliegt, umfasst, wobei die Zusammensetzung ferner einen oder mehrere Hilfsstoffe umfasst, ausgewählt aus der Gruppe, bestehend aus einem Schmiermittel, einem Gleitmittel, einem Antihaftmittel und einer Mischung aus zwei oder mehreren davon, wobei die Zusammensetzung eine feste Dosisform ist, und wobei eine Einzeldosis der Zusammensetzung 8 bis 14 Stunden lang für eine therapeutische Blut/Plasma-Konzentration von Phenylephrin in einem Individuum sorgt.

2. Die Zusammensetzung gemäß Anspruch 1, die ferner ein oder mehrere Wirkstoffe umfasst, ausgewählt aus der Gruppe, bestehend aus einem Antihistaminikum, einem Analgetikum, einem Antipyretikum und einem nichtsteroidalen Antiphlogistikum in einer Form mit sofortiger Freisetzung.

3. Die Zusammensetzung gemäß Anspruch 1, die ferner Loratadin oder Desloratadin in einer Form mit sofortiger Freisetzung umfasst.

4. Eine pharmazeutische Zusammensetzung mit verlängerter Freisetzung, die eine Zweischicht-Tablette umfasst, wobei eine Schicht aus einer Retard-Matrix besteht, die eine Mischung aus Hydroxypropylmethylcellulose, die in 20 bis 70 Gew.-% der festen Dosisform vorliegt, und Carboxymethylcellulose-Natriumsalz, das in 5 bis 50 Gew.-% der festen Dosisform vorliegt, umfasst, verabreicht mit Phenylephrin oder einem Salz davon, und die andere Schicht aus einer Schicht mit sofortiger Freisetzung besteht, die einen weiteren Wirkstoff enthält, ausgewählt aus einem oder mehreren von: einem Antihistaminikum, einem Analgetikum, einem Antipyretikum und einem nichtsteroidalen Antiphlogistikum, wobei eine einzelne verabreichte Dosis der Zusammensetzung 8 bis 14 Stunden lang für eine therapeutische Blut/Plasma-Konzentration von Phenylephrin in einem Individuum sorgt.

5. Die Zusammensetzung gemäß Anspruch 4, wobei der andere Wirkstoff Loratadin oder Desloratadin ist.

6. Die Zusammensetzung gemäß Anspruch 1, wobei das Phenylephrin oder ein pharmazeutisch annehmbares Salz in 5 bis 30 Gew.-% der festen Dosisform vorliegt.

7. Die Zusammensetzung gemäß Anspruch 1, wobei das Antihaftmittel Polyvinylpyrrolidon in einer Menge von 0,1 bis 10 Gew.-% der festen Dosisform ist.

8. Die Zusammensetzung gemäß Anspruch 7, wobei das Polyvinylpyrrolidon eine Partikelgrößenverteilung besitzt, bei der 90% der Partikel eine Größe von weniger als 20 Mikrometer und 99% der Partikel eine Größe von weniger als 400 Mikrometer besitzen.

9. Die Zusammensetzung gemäß Anspruch 7, wobei das Polyvinylpyrrolidon eine spezifische Oberfläche von 3 bis 6 Quadratmeter pro Gramm besitzt.

10. Die Zusammensetzung gemäß Anspruch 1, die ferner eine wasserlösliche Polymerfilmbeschichtung umfasst.

11. Die Zusammensetzung gemäß Anspruch 10, wobei der wasserlösliche Polymerfilm Loratadin oder Desloratadin enthält.

12. Ein Verfahren zur Herstellung einer Phenylephrin-Formulierung mit verlängerter Freisetzung, umfassend den Einbau von Phenylephrin oder eines pharmazeutisch annehmbaren Salzes davon in eine hydrophile Polymermatrix, wobei die hydrophile Polymermatrix eine Mischung aus Hydroxypropylmethylcellulose, die in 20 bis 70 Gew.-% der festen Dosisform vorliegt, und Carboxymethylcellulose-Natriumsalz, das in 5 bis 50 Gew.-% der festen Dosisform vorliegt, umfasst, das Kombinieren der hydrophilen Polymermatrix mit einem oder mehreren Hilfsstoffen, ausgewählt aus der Gruppe, bestehend aus einem Schmiermittel, einen Gleitmittel, einem Antihaftmittel und Mischungen aus zwei oder mehreren davon, und das Verpressen der resultierenden Mischung zu einer festen Dosisform, wobei eine einzelne Dosis der Formulierung mit verlängerter Freisetzung 8 bis 14 Stunden lang für eine therapeutische Blut/Plasma-Konzentration von Phenylephrin in einem Individuum sorgt.

## Revendications

1. Composition pharmaceutique à libération prolongée comprenant de la phényléphrine ou un sel pharmaceutiquement acceptable de celle-ci dans une matrice ou treillis polymère hydrophile, dans laquelle la matrice ou treillis polymère hydrophile comprend un mélange d'hydroxypropylméthylcellulose qui est présente en de 20 à 70 % en poids de la forme d'administration solide et de sel de sodium de carboxyméthylcellulose qui est présent en de 5 à 50 % en poids de la forme d'administration solide, où la composition comprend en outre un ou plusieurs excipients sélectionnés parmi le groupe d'un lubrifiant, un agent de glissement, un agent anti-adhérent et un mélange de deux ou plus de ceux-ci, où la composition est sous une forme d'administration solide et où une seule dose de la composition atteint une concentration thérapeutique de phényléphrine dans le sang/plasma chez un individu en l'espace de 8 à 14 heures.

2. Composition selon la revendication 1, comprenant en outre un ou plusieurs principes actifs sélectionnés parmi le groupe consistant en un antihistaminique, un analgésique, un antipyrétique et un agent anti-inflammatoire non stéroïdien sous forme de libération instantanée.

3. Composition selon la revendication 1, comprenant en outre de la loratadine ou de la desloratadine sous forme de libération instantanée.

4. Composition pharmaceutique à libération prolongée comprenant un comprimé bicouche, une couche constituée d'un matrice à libération prolongée comprenant un mélange d'hydroxypropylméthylcellulose qui est présente en de 20 à 70 % en poids de la forme d'administration solide et de sel de sodium de carboxyméthylcellulose qui est présent en de 5 à 50 % en poids de la forme d'administration solide, administrée avec de la phényléphrine ou un sel de celle-ci, et l'autre couche constituée d'une couche à libération instantanée contenant un autre principe actif sélectionné parmi un ou plusieurs d'entre un antihistaminique, un analgésique, un antipyrétique et un agent anti-inflammatoire non stéroïdien, où une seule dose administrée de la composition atteint une concentration thérapeutique de phényléphrine dans le sang/plasma chez un individu en l'espace de 8 à 14 heures.

5. Composition selon la revendication 4, dans laquelle l'autre principe actif est de la loratadine ou de la desloratadine.

6. Composition selon la revendication 1, dans laquelle la phényléphrine, ou un sel pharmaceutiquement acceptable de celle-ci, est présente en de 5 à 30 % en poids de la forme d'administration solide.

7. Composition selon la revendication 1, dans laquelle l'agent anti-adhérent est de la polyvinylpyrrolidone en une quantité de 0,1 à 10 % en poids de la forme d'administration solide.

8. Composition selon la revendication 7, dans laquelle la polyvinylpyrrolidone a à une distribution de taille de particules de 90 % de particules de moins de 20 microns et de 99 % de particules de moins de 400 microns.

9. Composition selon la revendication 7, dans laquelle la polyvinylpyrrolidone a une aire spécifique de 3 à 6 mètres carrés par gramme.

10. Composition selon la revendication 1, comprenant en outre un enrobage en film polymère soluble dans l'eau.

11. Composition selon la revendication 10, dans laquelle le film polymère soluble dans l'eau contient de la loratadine ou de la desloratadine.

12. Procédé de préparation d'une formulation à libération prolongée de phényléphrine comprenant incorporer de la phényléphrine ou un sel pharmaceutiquement acceptable de celle-ci, dans une matrice polymère hydrophile, où la matrice polymère hydrophile comprend un mélange d'hydroxypropylméthylcellulose qui est présente en de 20 à 70 % en poids de la forme d'administration solide et de sel de sodium de carboxyméthylcellulose qui est présent en de 5 à 50 % en poids de la forme d'administration solide, combiner la matrice polymère hydrophile avec un ou plusieurs excipients sélectionnés parmi le groupe d'un lubrifiant, un agent de glissement, un agent anti-adhérent et des mélanges de deux ou plus de ceux-ci, et comprimer-presser le mélange résultant en une forme d'administration solide, où une seule dose de la formulation à libération prolongée atteint une concentration thérapeutique de phényléphrine dans le sang/plasma chez un individu en l'espace de 8 à 14 heures.
